Europäisches Patentamt

European Patent Office

Office européen des brevets

(11) Publication number: **0 198 639**
**A1**

## EUROPEAN PATENT APPLICATION

(21) Application number: 86302526.8

(22) Date of filing: 04.04.86

(51) Int. Cl.⁴: **G 01 N 33/68,** G 01 N 33/545, G 01 N 33/547, G 01 N 33/535

(30) Priority: 04.04.85 JP 72109/85

(43) Date of publication of application: 22.10.86 Bulletin 86/43

(84) Designated Contracting States: DE FR SE

(71) Applicant: SHIONOGI & CO., LTD., 12, Dosho-machi 3-chome Higashi-ku, Osaka 541 (JP)

(72) Inventor: Uo, Junko, 79, Nakabayashi-cho Shugakuin, Sakyo-ku Kyoto-shi Kyoto (JP)
Inventor: Nishimura, Masaji, 53-14 Shunei-cho Saiin, Ukyo-ku Kyoto-shi Kyoto (JP)
Inventor: Horiguchi, Kazuo, 97 Ooi-cho Tanaka, Sakyo-ku Kyoto-shi Kyoto (JP)
Inventor: Nakanishi, Setsuko, Susumukogyo-ryo 3-16, Kutsukake-cho, Ooe, Nishikyo-ku Kyoto-shi, Kyoto (JP)
Inventor: Kohno, Takeshi, Daigoshida-danchi, 1-638 1, Nishinotobu Ishida, Fushimi-ku Kyoto-shi Kyoto (JP)

(74) Representative: Bizley, Richard Edward et al, BOULT, WADE & TENNANT 27 Furnival Street, London EC4A 1PQ (GB)

(54) The determination of albumin and a carrier and fixation technique usable therein.

(57) The use of albumin or anti-albumin antibody, in either case fixed to a low temperature plasma treated membrane carrier, in the determination of albumin enables such determinations to be conveniently, quickly and specifically performed with the albumin present in from trace to large amounts.

EP 0 198 639 A1

- 1 -

## THE DETERMINATION OF ALBUMIN AND A CARRIER AND FIXATION TECHNIQUE USABLE THEREIN

The total protein amount and the albumin/globulin ratio in serum bear a significant clinical importance in diagnoses of malnutrition, hepatic disorders, renal diseases, inflammatory diseases, autoimmune diseases, and so forth. It is well known that proteinuria is closely related with renal diseases, and research into the nature of renal diseases by protein analysis has become popular in recent years.

Although certain types of serum proteins pass through the renal glomeruli, more than 95% of the proteins filtered through the glomeruli are reabsorbed at the renal tubules. Proteinuria means that the amount of total protein excreted into the urine per day exceeds 100 - 150 mg (Ishimoto et al.: Sogo Rinsho, 27, 1223 - 1229, 1978). In a normal subject, albumin accounts for about 80% of the urinary proteins which mainly consist of proteins with a relatively small molecular weight among all serum proteins.

At present, proteinuria is classified into 2 types: one originating from the glomerulus and the other from the renal tubules. The urinary proteins of patients with glomerulonephritis or many other renal diseases consist mainly of albumin with a molecular weight of 67,000, whereas both proteins with molecular weight of 150,000 or more and those with molecular weight of 60,000 or less are scarce (glomerular proteinuria). In Fanconi's syndrome or renal tubular disorders due to heavy metal or drug intoxication, proteins filtered through the glomerulus are excreted into the urine without being reabsorbed. Daily total protein excreted into the urine is relatively small in amount and consists mainly of proteins with low molecular weights: less than 60,000 (renal tubular proteinurea).

Accordingly, the determination of albumin is essential for studies on the causes of diseases, especially diseases due to renal disorders, their therapies, and diagnoses, and has a significant clinical importance.

Conventional methods for the determination of albumin are roughly classified into two classes: methods to determine fractionated albumin (salting out methods, electrophoresis), and methods to determine specifically albumin alone (chromometry). Of the former, the history of the salting-out method is long, but it has scarcely been used because electrophoresis has become more practicable.

Chromometry is a method widely adopted in clinical fields; for example, the BCG Method (Dumas et al., Clin. Chim. Acta., 31, 87, 1971) is a method to determine the increase in absorbance at 630 nm, which is caused by bonding to albumin Bromcresol green (BCG) reagent which is dissolved in citric acid buffer solution at pH 4.2 containing nonionic surfactant Brij-35. Some simplified methods, such as Esbach's method, Sueyoshi's method, chromometry, and so on, are also clinically used.

A recently developed coomassie-brilliant blue-G250 method (Tonein-TP method Kit, Otsuka Assay) has made possible the determination of protein at the concentration of 10 - 100 mg/L, but this is the determination for total proteins in urine, not for albumin specifically. In the Japanese Patent Unexamined Publication No. 53-47517, a method for the determination of antigen protein by using a cellulose membrane was disclosed, but the cellulose membrane is difficult to handle and is poor in reproducibility. In another method for determination using anti-human albumin antibody-sensitized red blood cells or latex particles (Japanese Patent Unexamined Publication No. 54-80410, Japanese Patent Unexamined Publication No. 55-30654), the dilution ratios of samples are so wide that significant errors might occur.

Fractionation by electrophoresis has a high separation capacity, but the dyes used for detection of the fraction bands have varied in absorption capacity depending upon the types of proteins, and have problems in determination accuracy. Furthermore, even if a simplified cellulose acetate membrane is used, handling is so complicated that it requires extremely proficient techniques; besides, the determination takes a long time.

In the case of the BCG method, since there is a difference in the time-course of coloration between an albumin standard solution and albumin contained in serum or other vital samples (in the latter the coloration is gradually intensified, and the higher the temperature rises the stronger the coloration becomes), deviations in the determination time and in the temperature lead to large errors. Moreover, $\gamma$-globulin also shows 2 - 3% of cross reactivity and, therefore, this can not be regarded as a qualitative and quantitative determination specific to albumin (Doumas et al., Clin. Chim. Acta., 31, 87, 1971). In addition, since the lower limit of detection of the BCG method is 20g/L, it can not be applied to the detection of albumin in urine.

On the other hand, the lower limites for detection of the simplified methods such as Esbach's method, Sueyoshi's method, colorimetry etc., are only 300 - 500 mg/L but the reactions by these methods are hardly specific to albumin.

Accordingly, simplified, accurate and economical determination methods, which have sufficient sensitivity for determinations without diluting or condensing the samples, have long been awaited.

The invention provides the use of albumin or anti-albumin antibody, in either case fixed to a low temperature plasma treated membrane carrier, in the determination of albumin.

This invention relates to the determination of albumin by means of an enzyme immunoassay employing a hydrophilic membrane carrier treated with low temperatures plasma (Japanese Patent Application No. 58-199856; Japanese Patent Application No. 59-277656). Thus, anti-albumin antibody or albumin may be made to contact and be fixed with a membrane carrier into which a certain amount of aldehyde groups have been introduced. Then various kinds of immuno-reactions are made to occur between the carrier and the albumin or anti-albumin antibody which is conjugated with an enzyme "label" such as glucose oxidase (GOD) and horesradish peroxidase (HRP) for measuring the enzyme activity colorimetrically or electrical output is used for the determination of albumin. The present invention enables the determination of albumin conveniently and quickly and the assay specifically of albumin contained in samples in trace to large amounts by making use of the specificity of the antigen-antibody reaction. Thus, in case of the electical output, albumin in samples can be specifically determined with a sensitivity of 0.5 - 160 mg/L; in case of colorimetry, 0.03 - 4mg/L, with a reaction time ranging from 10 minutes to 3 hours in both methods. Accordingly, the present method can provide important information not only for diagnoses of various diseases, especially renal disease, but also for prevention, early diagnosis, and prognosis.

In the accompanying drawings:-

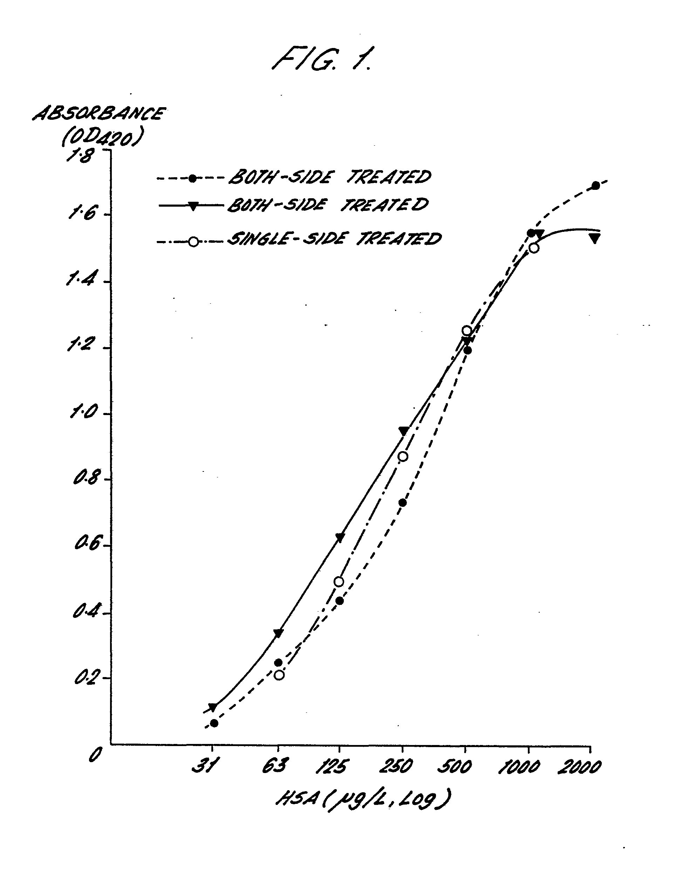

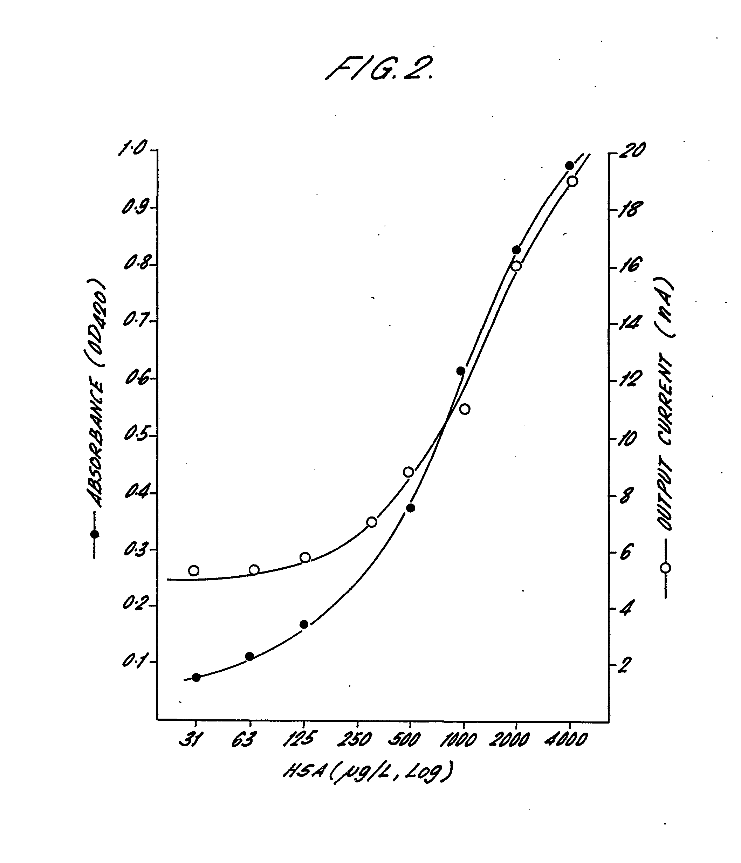

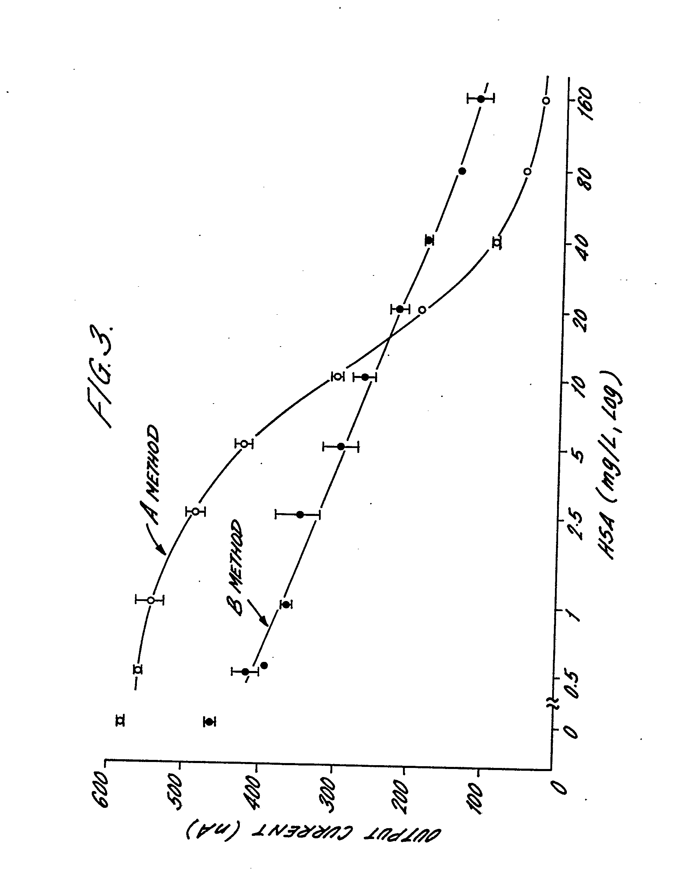

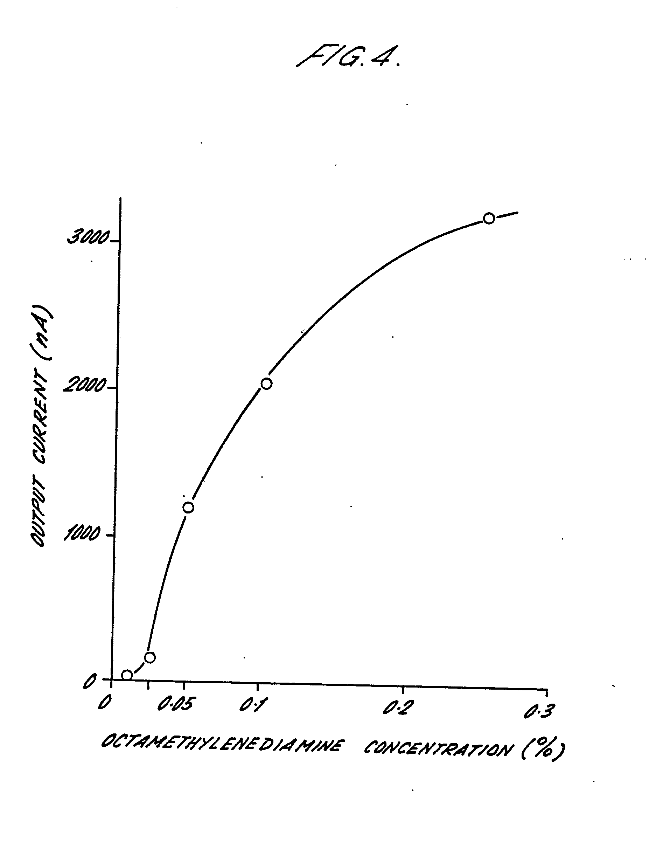

Fig. 1 shows the results of Example 1 (see after). The ordinate represents the absorbance at 420nm, while the abscissa the concentrations of HSA. ...●.,. shows the results of the both-side treated membrane in 60 minutes of albumin reaction time; — ▼ — those of the both-sides treated-membrane in 3 hours of albumin reaction in time; and — — ◯ — — those of the single-side treated membrane in 60 minutes of albumin reaction time. Fig. 2 shows the results of Example 3 (see after). The ordinate represents the absorbance at 420 nm and the output current, while the abscissa, the HSA concentration. Fig. 3 shows the results of Example 4 (see after). The ordinate indicates the output current, while the abscissa, the HSA concentration. Fig. 4 shows the results of Example 7. The ordinate represents the output current, while the abscissa, the octamethylenediamine concentrations.

The present invention has been effected after great efforts to develop a new method for determining albumin contained in medium or serum, especially in urine, accurately, conveniently and economically.

1) Treatment of the membrane carrier

In the same way as described in Japanese Patent Application No. 58-199856 and Japanese Patent Application No. 59-277656, porous polyolefin membranes etc. are treated with acetone, depending on necessity, and then treated with low temperature plasma to be made hydrophilic. The treatment with low temperature plasma may be performed on the whole or a part of the surface, and may be performed on both sides or one side of the membrane. Next, said membrane is treated with 0.02 - 10% aqueous octamethylenediamine solution. Depending upon the concentration of aqueous octamethylenediamine solution, any desired amount of protein can be combined with the membrane. With concentrations lower than the above-mentioned level, protein scarcely combines with the membrane, whilst with concentrations higher than the above level, the amount of protein to be combined with the membrane is saturated. Next, after the membrane is washed with phosphate buffer solution (hereafter abbreviated as PB), phosphate buffered saline (hereafter abbreviated as PBS) and so forth, aldehyde groups are introduced into the membrane with glutaraldehyde and so on. After washing with PB, PBS etc., the membrane, the whole surface of which is treated with low-temperature plasma, is cut to pieces of 0.5 - 15 mm diameter.

2) Fixation of anti-albumin antibody or albumin

Anti-albumin antibody or albumin is fixed on the membrane carrier treated in the above mentioned manner. The anti-albumin antibody can be obtained by the following method: Highly purified albumin containing no globulin, together with an adjuvant, is injected several times into animals such as rabbits,goats, and guinea pigs. Then, blood samples are collected to prepare antisera, to which antiseptics are added for storage, and used as anti-albumin antibody. Commercial anti-albumin antibody may also be used. With PBS and so on, the anti-albumin antibody should be diluted 1,500 to 3,000 times when determined colorimetrically; it should be diluted 100 - 400 times when determined by electrodes. Albumin should be diluted to 0.001 - 4 $\mu g/\mu l$, and more suitably to 0.05 - 0.2 $\mu g/\mu l$. When the diluted solution is dropped onto the membrane, 5 - 100 $\mu l$, more suitably 10 - 20 $\mu l$, of the solution is used. When the membrane is steeped in the diluted solution, 100 - 1,000 $\mu l$, more suitably 30 - 400 $\mu l$, of it is used. The membranes are allowed to stand for 10 minutes to 48 hours, more suitably for 5 - 24 hours, at 4 - 30°C for fixation of anti-albumin antibody or albumin. The amount of anti-albumin antibody or albumin to be fixed may be an amount necessary

0198639

- 8 -

for the determination of albumin. After washing with PB, PBS, etc., the membrane carrier is treated with the normal serum of a goat. rabbit, guinea pig, etc. or bovine serum albumin for blocking unreacted groups on it, and is washed with PB, PBS, and so forth.

3) Immunological reactions

On the membrane treated as mentioned above, the following immunological reactions are made to occur.

a. Sandwich method

Albumin solution of 50 - 500 $\mu$ l to be examined is contacted with the membrane carrier having the fixed anti-albumin antibody and is allowed to undergo immunological reaction for 10 minutes to 48 hours, preferably for 30 minutes to 3 hours, at 4 to 30°C, preferably at 25°C. Next, or simultaneously, an enzyme-conjugated anti-albumin antibody, diluted 10 - 3,000 times with PBS etc., containing surfactant such as Tween (Trade Mark), is added to undergo immunological reaction for 10 minutes to 2 hours, preferably for 30 to 60 minutes, at 4 - 37°C, preferably at 30 to 35°C. Recommended enzymes used on this occasion include horseradish peroxidase, glucose oxidase, $\beta$ -D-galactosidase, glucose-6-phosphate dehydrogenase, etc. The enzyme-conjugated anti-albumin antibody can be prepared by conventional methods such the glutaraldehyde method using glutaraldehyde as a cross-linking agent to bond the enzyme to the antibody; the maleimide method using maleimide as a cross-linking agent; and so on.

b. Competitive method

A solution of albumin to be examined, which is dissolved in PBS containing surfactant such as Tween etc., and a solution of enzyme-conjugated albumin are mixed in a predetermined ratio. The mixed solution is made to react with the membrane carrier having the fixed anti-albumin antibody for 10 minutes to 48 hours at 4 to 35°C; preferably for 30 to 60 minutes at 20 to 30°C. The methods for the preparation of the enzyme and the enzyme-conjugated albumin, which are used here, are the same as employed in the above-mentioned sandwich method.

c. Competitive inhibition method

Albumin to be examined is contacted with the membrane carrier having the fixed albumin. Then an enzyme-conjugated anti-albumin antibody is added to induce an immunological reaction. The reaction conditions, enzymes, and the preparation of enzyme-conjugated anti-albumin antibody are the same as those employed in the above-mentioned sandwich method.

A variety of immunological reactions other than those mentioned above may be applicable.

4) Detection

The membrane carrier where an immunological reaction has been made to occur, as mentioned above, is washed with PBS, PB etc., and subjected to the detection of bonded enzymes.

a. Colorimetry

- 10 -

A color-producing solution containing color formers such as ABTS, diphenylamine etc. is contacted with the membrane carrier. When ABTS-$H_2O_2$ color-producing solution is used, it is sufficient to react 100 - 500 $\mu$l of it with the carrier for 20 - 40 minutes at 30 - 35°C. Enzyme reaction inhibitors such as oxalic acid, sodium azide etc. are added to inhibit the reaction and the absorbance is determined.

b. Electric output determination method

Said membrane carrier is fixed to hydrogen peroxide electrodes, which are steeped into a substrate solution dissolved in PBS etc. When equilibrium is reached in about 30 seconds - 1 minute, the electrical output is determined.

The present invention removes the uncertainty of the prior art mentioned above, and enables the determination of albumin conveniently and quickly and the assay specifically of albumin contained in samples in trace to large amounts by making use of the specificity of the antigen-antibody reaction. In the case of the electrode method, albumin in samples can be specifically determined with a sensitivity of 0.5 - 160 mg/L; and in case of colorimetry, 0.03-4mg/L, with a reaction time ranging from 10 minutes to 3 hours in both methods. Accordingly, the present method can provide important information not only for diagnoses of various diseases, especially of renal diseases, but also for prevention, early diagnosis, and prognosis.

- 11 -

In the following Examples several preferred embodiments of the present invention are described. However, it is to be understood that the present invention is not intended to be limited to the specific embodiments.

Example 1

Microporous polypropylene film (Juragard #3400, 25 $\mu$ m thick, 0.02 x 0.2 $\mu$ m of the maximum aperture, hydrophilic, Polyplastics Co., Ltd.,) was treated with acetone, and one side of the membrane was treated for 30 min. with low temperature plasma or both sides of the membrane were treated for 20 minutes on each side. The treated membrane was steeped into 10% of aqueous octamethylenediamine solution and washed with water. Then it was steeped into 25% glutaraldehyde solution. After being washed with water, the membrane was cut into pieces of 13 $\phi$. Each of the obtained membrane pieces were put into each of Nunc 24 Well multidish (the diameter of well: about 16 mm, Nunc), to which 300 $\mu$ l of 3000 times-diluted goat anti-human serum albumin antiserum (Cappel) was added, and allowed to stand for 3-6 hours at 4°C. After the added antiserum was removed, the membrane piece was washed with 0.1M of phosphate buffer solution (hereafter abbreviated as PB) in pH 7.3. Then, normal goat serum (diluted 3000 times) was added to it and allowed to stand overnight at 4°C. After removal of the serum, 500 $\mu$ l of human serum albumin (hereafter abbreviated as HSA) (31-2000 $\mu$ g/L, 7 steps multiple dilution) was added as a standard solution to react with the membrane for 30 and 60 minutes when its both sides were treated with plasma, and for 60 minutes at 30°C when only one of the sides was treated. After the added standard HSA was removed, the membrane piece was washed with PB (pH 7.3). Then, 300 $\mu$ l of 3 ,000 times diluted solution of goat anti HSA antibody (IgG fraction, Cappel) conjugated with horseradish peroxidase (hereafter abbreviated as HRP), was added. Double-side-plasma-treated membranes were made to react for 30 minutes at 30°C , while single-side-treated ones for 60 minutes. After removing the goat anti HSA antibody solution and washing with PB, 400 $\mu$ l of ABTS [(2,2'-Azino-bis(3-ethylbenzthiazoline-6'-sulphonic acid)]-$H_2O_2$ color forming solution was added to react for 30 minutes at 30°C. The reaction was stopped by adding 2 ml of 0.1M oxalic acid for determination of the absorbance at 420 nm. The results are shown in Fig. 1. By the sandwich-colorimetry, 0.03 -4 mg/L of albumin can be determined.

Example 2

In the same way as that employed in Example 1, standard curves were prepared using the standard HSA (31 - 2,000 $\mu$ g/L, 7 steps multiple dilution) for determination of the concentration of albumin in human urine samples. In this case, in addition to the method of Example 1 (two steps-sandwich method), another method, the HRP-conjugated anti HSA antibody was made to react simultaneously either with the standard HSA or with urine sample (one-step sandwich method), was also performed. The results are shown in Table 1.

Table 1. HSA in urine (Voluntary urination by a healthy male)

| Samples | Ages | Ashing treatment | Sandwich method | Dilution* steps | HSA mg/L $\pm$ S.D. |
|---------|------|------------------|-----------------|-----------------|---------------------|
| A | 36 | Both sides | 2 step | 5 | 1.98 $\pm$ 0.21 |
|   |    | " | 1 step | 5 | 2.16 $\pm$ 0.16 |
| B | 36 | " | 2 step | 2 | 2.60 |
|   |    | " | 1 step | 2 | 2.60 |
| C | 28 | Single side | 2 step | 2 | 2.60 |
|   |    | " | 1 step | 2 | 3.08 |
| D | 33 | " | 2 step | 6 | 8.48 $\pm$ 0.89 |
|   |    | " | 1 step | 6 | 8.27 $\pm$ 0.96 |

* Multiple dilution up to 2 - 64 times, determined 3 times in each step.

As clear from Table 1, both 1-step sandwich method and 2-steps sandwich method show similar value. Ashing treatment may be performed on the both sides or one side.

Example 3

Similarly to Example 1, both sides of microporous

- 14 -

polypropylene film (Juragard #3400, Polyplastics Co., Ltd.) were treated with low temperature plasma and cut into 13ф. As enzyme-conjugated antibody, glucose oxidase (hereafter abbreviated as GOD)-rabbit anti-HSA-Fab' was used; as antiserum to be fixed, 1,000 times diluted solution of rabbit anti HSA antiserum; and as a blocking agent, rabbit normal serum (300 times diluted). Enzyme immuno reaction was performed similarly to Example 1. For each of the standard HSA (31 - 4,000 $\mu$g/L) 8-steps multiple diluted solutions, 6 sheets of the membrane carrier were used. Three of them were used for colorimetry by ABTS-HRP color forming solution. The remaining 3 sheets were settled to the hydrogen peroxide electrodes (2ф, platinum), which were steeped into 15 ml of phosphate buffered saline (hereafter abbreviated as PBS) containing 50 mg of glucose as a substrate for measurement of the output current. The results of the colorimetry and electrode methods are shown in Fig. 2

Example 4

Microporous polypropylene film (Juragard #2500, 25$\mu$m thick, the maximum aperture: 0.04 x 0.4 $\mu$m, hydrophilic, Polyplastics Co., Ltd.) was cut into pieces 150 mm x 150 mm in size. One piece was placed between two similar size pieces of aluminium shielding covers with perforations (6 mm diameter, 36 holes drilled), and both the sides were treated with steam plasma (100 mA, 500 V) for 3 minutes each to give hydrophilic spot membranes. Five to 10 $\mu$l

of 0.3% octamethylenediamine was dropped on each spot. When the whole of a spot was wet to become translucent, the excess of water was wiped away with filter-paper. Next, the membrane was steeped into 5% glutaraldehyde solution and was washed with distilled water or PBS.

Method A (Competitive method)

On each spot, 20 μl of rabbit anti HSA antiserum 200 times diluted solution was dropped and the spots were left overnight at 4°C. After removal of the antiserum and washing with PBS, 20 μl of normal rabbit serum or 20 μl PBS containing containing bovine serum albumin (hereafter abbreviated as BSA) was dropped on the spot and left for 6 hours at 4°C. After removal of the added PBS, the spot was washed with PBS. Twenty microliters of the mixed solution of 150 μl GOD-HSA and 150 μl standard HSA was dropped on the spot and left for one hour at 25°C. After being removed from the mixed solution and washed with PBS, the membrane was fixed to the hydrogen peroxide electrodes (4φ, platinum), which was steeped into 15 ml of PBS containing 50 mg of glucose for determining the output current.

Method B (Competitive inhibition method)

Twenty microliters of PBS containing 2 μg of HSA was dropped on each spot and left overnight at 4°C. After removal of added PBS and washing with PBS, 20 μl of normal rabbit serum or 20 μl of

BSA-PBS was dropped and left for 6 hours at 4°C. After removal of added PBS and washing with PBS, 8 $\mu$1 of HSA was added. Two minutes later 10 $\mu$1 of GOD-Fab' (from rabbit anti HSA-IgG ) 30 times diluted solution was added and left for one hour at 25°C. After washing with PBS, the output current was measured with the electrodes in a way similar to Method A.

The results are shown in Fig.3. Accurate measurement of HSA can be achieved by both A and B methods in HSA concentrations of 0.5 - 160 mg/L.

Example 5

By the colorimetry according to the 2 steps sandwich method of Example 1, and Method A and B in Example 4, urinary albumin was determined. At the same time, an addition recovery test was carried out. The addition recovery test is one where a certain amount of albumin is added to original urine for determination. The addition recovery rate is expressed as follows:

Actual measurement value/theoretical value (%).

Standard HSA: 1 - 80 mg/L (8 steps multiple dilution) Sample: Original urine (Method A and B) and diluted solution of original urine (sandwich method). The original urine, supplemented 10 mg or 40 mg of HSA (for addition recovery test).

In the sandwich colorimetry, measurement was performed twice each for 4 steps multiple dilution; 6 times in Method A and B; and 3

- 17 -

times each for 2 steps multiple dilution in the recovery test. The results are shown in Table 2.

## Table 2

| Samples | Sandwich colorimetry (mg/L) | A method (mg/L) | B method (mg/L) | HSA addition recover rate (%) | |
|---|---|---|---|---|---|
| | | | | A method | B method |
| Male aged 29 | 14.0 | 13.3±0.9 | 13.1±4.5 | 110.9±7.6 | 113.0±4.6 |
| Male aged 37 | 19.1 | 16.4±0.2 | 18.5±0.5 | 96.7±5.1 | 108.6±6.4 |

Example 6

According to Method A similar to Example 4, blood albumin was determined. At the same time, an determination based upon the conventional BCG method was also performed for comparison.

Standard HSA: 1 - 80 mg/L, 8 step multiple dilution Samples: method A; 1000, 3000 and 10000 times diluted serum [PBS-Tween (Trade Mark)]

BCG method: Original serum

The results are shown in Table 3.

Table 3

| Samples | Method A | BCG method |
|---|---|---|
| Female aged 43 | 4.4 g/dl | 4.5 g/dl |
| Female aged 38 | 5.0 g/dl | 4.9 g/dl |
| Female aged 58 | 5.3 g/dl | 5.3 g/dl |

As obvious from Table 3, the method of this invention is quite consistent with BCG method, and can be sufficiently applied to the determination of serum albumin.

Example 7

Microporous polypropylene film (Juragard #2500, Polyplastics Co., Ltd., ) was treated with low temperature plasma in the same way as employed in Example 4. Five to 10 $\mu$l each of various concentrations of octamethylenediamine (5, 1, 0.25, 0.1, 0.05, 0.025 and 0.0125%) was dropped on each spot. It was air dried, or an excess of water was wiped away with filter-paper when the whole of the spot was wet to become translucent. After being steeped into 5% glutaraldehyde solution, the spot was washed with distilled water or PBS. Each concentration of aqueous GOD solution (1, 2 and 5 $\mu$g/50$\mu$l/spot) was dropped on each spot and was preserved for 18 hours at 4°C in a plastic box where filter papers wetted with water was spread. Twenty $\mu$ l of water was removed from the drop on each spot (the residue) to be put into 10 ml of PBS (containing 50 mg of glucose) and the output current was measured using the hydrogen peroxide electrodes (4$\phi$, platinum). The results are indicated in Fig. 4. When octamethylenediamine concentration is less than 0.25%, GOD amount fixed to the electrode are proportional to the concentrations of octamethylenediamine, but when the concentration is over 0.25%, saturated state is reached. Furthermore, when albumin, anti-albumin antiserum etc. are fixed, it has been proved that an almost equal amount to the mass protein of GOD is fixed. That is, any desired amount of protein can be fixed by adjusting the concentration of octamethylenediamine.

CLAIMS :

1. The use of albumin or anti-albumin antibody, in either case fixed to a low temperature plasma treated membrane carrier, in the determination of albumin.

2. A method for the determination of albumin comprising fixing a predetermined amount of albumin or anti-albumin antibody to a membrane carrier treated with low temperature plasma: and in the case of fixed antibody either contacting said anti-albumin antibody with albumin and then or simultaneously with enzyme-conjugated anti-albumin antibody and reacting the combined enzyme with a substrate for measurement or contacting said anti-albumin antibody with albumin and enzyme-conjugated albumin and reacting the combined enzyme with a substrate for measurement; and in the case of fixed albumin contacting said albumin with albumin and then with enzyme-conjugated anti-albumin antibody and reacting the combined enzyme with a substrate for measurement.

3. A method as claimed in Claim 2, wherein said membrane carrier is a porous polyolefin membrane carrier.

4. A method as claimed in Claim 3, wherein said polyolefin is polypropylene.

5. A method as claimed in any one of Claims 2 to 4, wherein said enzyme is glucose oxidase or horseradish peroixdase.

6. A method as claimed in any one of Claims 2 to 5, wherein said measurement is performed with colorimetry or is electrochemically performed by connecting said membrane carrier to an electrode for enzyme activity detection.

7.   A membrane carrier for the determination of albumin prepared by fixing albumin or anti-albumin antibody to a porous polyolefin membrane treated with low temperature plasma.

8.   A fixation method comprising treating a porous polyolefin membrane, treated with low temperature plasma, with an aqueous octamethylenediamine solution and fixing to said membrane a predetermined amount of protein according to the concentration of said aqueous octamethylenediamine solution.

9.   A carrier as claimed in Claim 7 or a method as claimed in Claim 8, wherein said polyolefin is polypropylene.

0198639

1/4

FIG. 1.

FIG. 2.

0198639

2/4

FIG. 3.

*A METHOD*

*B METHOD*

OUTPUT CURRENT (nA)

HSA (mg/L, Log)

4/4

FIG. 4.

0198639

European Patent
Office

**EUROPEAN SEARCH REPORT**

Application number

EP 86 30 2526

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | CLINICAL CHEMISTRY, vol. 29, no. 2, 1983, pages 355-357; B.A. FIELDING et al.: "Enzyme immunoassay for urinary albumin" * Page 355, column 1, line 16 - page 356, column 1, line 43; page 356, figure 1 * | 1,2,5, 6 | G 01 N 33/68 G 01 N 33/545 G 01 N 33/547 G 01 N 33/535 |
| A | EP-A-0 101 912 (GENETIC DIAGNOSTIC CORP.) * Page 1, line 1 - page 2, lin3e 24; page 6, lines 1-7 * | 2,5,6 | |
| A,D | PATENTS ABSTRACTS OF JAPAN, vol. 2, no. 89 (C-78)[1352], 28th April 1978; & JP - A - 53 47 517 (NIPPON CHEMIPHER K.K.) 28-04-1978 * Whole document * | 1-6 | |
| | | | TECHNICAL FIELDS SEARCHED (Int. Cl.4) |
| A | CHEMICAL ABSTRACTS, vol. 102, no. 26, 1st July 1985, page 78, abstract no. 222209p, Columbus, Ohio, US; & JP - A - 60 25 733 (TOYOTA MOTOR CO. LTD.) 08-02-1985 * Whole document * | 1,3,4 | G 01 N |
| | --- -/- | | |

The present search report has been drawn up for all claims

| Place of search | Date of completion of the search | Examiner |
|---|---|---|
| THE HAGUE | 09-07-1986 | HITCHEN C.E. |

# EUROPEAN SEARCH REPORT

European Patent Office

Application number

EP  86 30 2526

## DOCUMENTS CONSIDERED TO BE RELEVANT

Page 2

| Category | Citation of document with indication, where appropriate, of relevant passages | Relevant to claim | CLASSIFICATION OF THE APPLICATION (Int. Cl.4) |
|---|---|---|---|
| A | CHEMICAL ABSTRACTS, vol. 99, no. 1, 4th July 1983, page 241, abstract no. 2339k, Columbus, Ohio, US; D. PETROVIC et al.: "Immobilization of glucose isomerase. I. Covalent bonding of the enzyme to modified cellulose matrices", & GLAS. HEM. DRUS. BEOGRAD 1982, 47(10), 557-62 * Whole document * | 8 | |

-----

TECHNICAL FIELDS SEARCHED (Int. Cl.4)

The present search report has been drawn up for all claims

| Place of search | Date of completion of the search | Examiner |
|---|---|---|
| THE HAGUE | 09-07-1986 | HITCHEN C.E. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82